(19) Europäisches Patentamt
European Patent Office
Office européen des brevets

(11) Veröffentlichungsnummer: **0 221 439**
**A1**

(12) **EUROPÄISCHE PATENTANMELDUNG**

(21) Anmeldenummer: **86114577.9**

(22) Anmeldetag: **21.10.86**

(51) Int. Cl.4: **A01N 47/38**, C07D 211/60, C07D 211/62, C07D 211/64, C07D 265/30, C07D 279/12, C07D 405/04

(30) Priorität: **26.10.85 DE 3538097**
**14.02.86 DE 3604645**

(43) Veröffentlichungstag der Anmeldung:
**13.05.87 Patentblatt 87/20**

(84) Benannte Vertragsstaaten:
**AT BE CH DE ES FR GB IT LI NL SE**

(71) Anmelder: **HOECHST AKTIENGESELLSCHAFT**
**Postfach 80 03 20**
**D-6230 Frankfurt am Main 80(DE)**

(72) Erfinder: **Liebl, Rainer, Dr.**
**An der Weinleite 5b**
**D-8901 Todtenweis(DE)**
Erfinder: **Handte, Reinhard, Dr.**
**Theilweg 23**
**D-8901 Gablingen(DE)**
Erfinder: **Mildenberger, Hilmar, Dr.**
**Fasanenstrasse 24**
**D-6233 Kelkheim (Taunus)(DE)**
Erfinder: **Bauer, Klaus, Dr.**
**Kolpingstrasse 7**
**D-6054 Rodgau(DE)**
Erfinder: **Bieringer, Hermann, Dr.**
**Eichenweg 26**
**D-6239 Eppstein/Taunus(DE)**

(54) **Herbizide Mittel, die stickstoffhaltige Heterocyclen mit Arylcarbamoyl-Resten enthalten, sowie neue heterocyclische Derivate mit Arylcarbamoyl-Resten.**

(57) Gegenstand der Erfindung sind herbizide Mittel, die eine Verbindung der Formel I

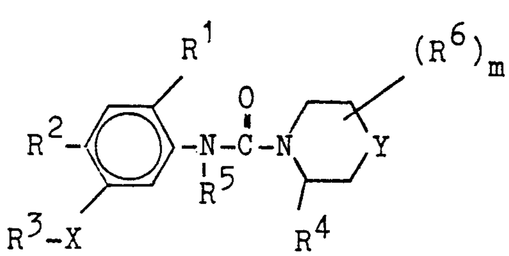

(I)

worin
X= O, S oder eine direkte Bindung, $R^1$= Wasserstoff, Fluor oder Chlor, $R^2$= Halogen, (subst.) Alkyl, (subst.) Alkoxy $R^3$= Wasserstoff, (subst.)Alkyl, Cycloalkyl, Alkenyl, Cycloalkenyl, Alkinyl, (subst.)Phenyl, (subst.)Benzyl, subst. Carbonyl, $R^4$= -CN, $-CONR^7R^8$, $-C(NOH)NH_2$, $-CO(NR^7-NR^9R^{10})$, $-CH-(ZR^{11})_2$ oder $-CO-ZR^3$; Y= $C(R^{12})_2$ S,

EP 0 221 439 A1

SO, $SO_2$ oder $-NR^{13}-$, $R^5$ = H, (subst.)Alkyl, $R^6$ = Alkyl, (subst.)Alkoxycarbonyl, (subst.) Phenyl oder Benzyl oder deren in der Landwirtschaft einsetzbaren Salze enthalten. Einige dieser Verbindungen sind neu und werden ebenfalls von der Erfindung umfaßt.

# Herbizide Mittel, die stickstoffhaltige Heterocyclen mit Arylcarbamoyl-Resten enthalten, sowie neue heterocyclische Derivate mit Arylcarbamoyl-Resten

Es wurde überraschenderweise gefunden, daß bestimmte heterocyclische Carbonsäuren und ihre Derivate, die 1-(N-Arylcarbamoyl)-Reste enthalten, eine ausgezeichnete herbizide Wirkung aufweisen. Einige dieser Verbindungen werden in EP-A 70 389 als Zwischenstufen bei der Herstellung von 1,3-Diazabicyclo[4.3.0]-nonadionen beschrieben. Über eine biologische Wirksamkeit dieser Verbindungen wurde jedoch nicht berichtet. Ferner sind aus EP-A-104 532 einige weitere Verbindungen dieses Typs und deren Verwendung als Herbizide bekannt. Diese besitzen jedoch unzureichende Wirksamkeiten.

Gegenstand der Erfindung sind daher herbizide Mittel, die eine Verbindung der Formel I oder deren Salze

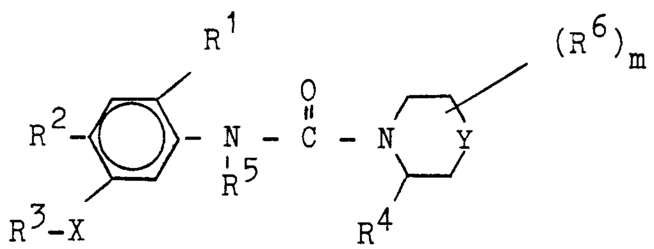

(I),

worin

X = Sauerstoff, Schwefel oder eine direkte Bindung

$R^1$ = Wasserstoff, Fluor oder Chlor,

$R^2$ = Fluor, Chlor, Brom, $(C_1\text{-}C_4)$Alkyl, das bis zu 6-fach durch Fluor und/oder Chlor substituiert sein kann, oder $(C_1\text{-}C_4)$Alkoxy, das bis zu 6-fach durch Fluor und/oder Chlor substituiert sein kann

$R^3$ = Wasserstoff, $(C_1\text{-}C_4)$Alkyl, $(C_3\text{-}C_6)$Cycloalkyl, $(C_3\text{-}C_6)$Alkenyl, $(C_5\text{-}C_6)$Cycloalkenyl, $(C_3\text{-}C_6)$-Alkinyl, $(C_1\text{-}C_4)$-Alkoxycarbonyl, $(C_1\text{-}C_4)$Alkoxycarbonyl$(C_1\text{-}C_4)$-alkoxycarbonyl, $(C_3\text{-}C_4)$Alkinyloxycarbonyl$(C_1\text{-}C_4)$-alkoxycarbonyl, $(C_1\text{-}C_4)$Alkoxy$(C_1\text{-}C_4)$alkoxycarbonyl-$(C_1\text{-}C_4)$alkoxycarbonyl, Trifluorethoxycarbonyl, Trifluorethoxycarbonyl$(C_1\text{-}C_4)$alkoxycarbonyl, $(C_1\text{-}C_4)$Alkoxy$(C_1\text{-}C_4)$alkyl, $(C_1\text{-}C_4)$-Alkoxy-carbonyl $(C_1\text{-}C_4)$alkyl, $(C_1\text{-}C_4)$Alkoxythiocarbonyl$(C_1\text{-}C_4)$alkyl, Phenyl oder Benzyl, die beide bis zu dreifach im Phenylring durch Halogen, $(C_1\text{-}C_4)$Alkyl, $(C_1\text{-}C_4)$-Alkoxy, $(C_1\text{-}C_4)$-Alkoxy-carbonyl, $CF_3$, $NO_2$ oder CN substituiert sein können,

$R^4$ = $-CN$, 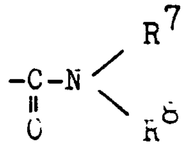, 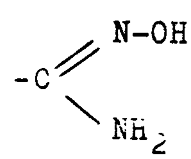, $-CO-ZR^5$,

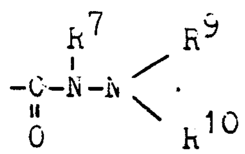 oder 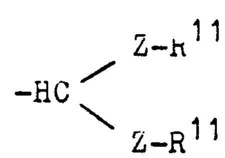

Y = $-O-$, 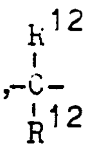, $-S-$, 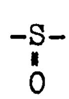, 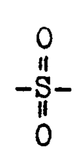 oder 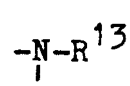

Z = Sauerstoff oder Schwefel,
$R^5$ Wasserstoff, $(C_1-C_4)$Alkyl, $(C_1-C_4)$Alkoxycarbonyl-$(C_1-C_4)$alkyl, oder Hydroxymethyl,
$R^6$ $(C_1-C_4)$Alkyl, $(C_1-C_4)$Alkoxycarbonyl, $(C_1-C_4)$Alkoxycarbonyl$(C_1-C_4)$alkoxycarbonyl, Phenyl oder Benzyl, die beide bis zu 3-fach durch Halogen, $(C_1-C_4)$Alkyl, $(C_1-C_4)$Alkoxy, $CF_3$ oder $NO_2$ substituiert sein können, $R^7$,$R^8$ unabhängig voneinander Wasserstoff, $(C_1-C_4)$-Alkoxy, Benzyloxy, $(C_1-C_4)$-Alkyl, $(C_3-C_6)$Cycloalkyl, $(C_3-C_6)$Alkenyl, $(C_5-C_6)$Cycloalkenyl, $(C_3-C_6)$Alkinyl, Phenyl oder Benzyl, die beide im Phenylring ein-bis dreifach durch Halogen, $(C_1-C_4)$Alkyl, $(C_1-C_4)$-Alkoxy, $(C_1-C_4)$-Alkoxy-carbonyl, $CF_3$, CN oder $NO_2$ substituiert sein können, oder
$R^7$ und $R^8$ zusammen mit dem Stickstoffatom, an das sie gebunden sind, einen fünf-bis sechsgliedrigen, gesättigten, gegebenenfalls bis zu vierfach durch $(C_1-C_4)$Alkyl substituierten, heterocyclischen Ring bilden, der ein oder mehrere Ringglieder aus der Gruppe O, S, $NR^{14}$, wobei $R^{14}$ Wasserstoff, $(C_1-C_4)$-Alkyl, Phenyl oder Benzyl, die beide ein-bis dreifach durch Halogen, $CF_3$, $(C_1-C_4)$-Alkyl oder $(C_1-C_4)$-Alkoxy substituiert sein können, bedeutet, enthält und worin zusätzlich eine Methylengruppe durch C=O ersetzt sein kann, und der Ring zusätzlich einen ankondensierten Benzolring oder einen ankondensierten 5-bis 6-gliedrigen gesättigten Kohlenwasserstoffring aufweisen kann,
$R^9$, $R^{10}$ unabhängig voneinander Wasserstoff, $(C_1-C_4)$Alkyl, Phenyl oder Benzyl, die beide bis zu 3-fach durch $(C_1-C_4)$Alkyl, Halogen, $CF_3$, $NO_2$ oder $(C_1-C_4)$Alkoxy substituiert sein können; $(C_1-C_4)$Alkoxycarbonyl; oder einer der Reste $R^9$ oder $R^{10}$ einen Rest der Formeln $-CO-R^{15}$, $-CO-NHR^{15}$,
$-S(O)_nR^{15}$ oder $-\underset{Z}{\overset{\|}{P}}(ZR^{15})_2$ oder
$R^9$ und $R^{10}$ zusammen die Gruppe $=C(R^{15})_2$,
$R^{11}$ jeweils $(C_1-C_4)$Alkyl oder zwei Reste $R^{11}$ zusammen mit dem Rest

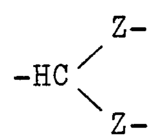

an den sie gebunden sind, einen 5-oder 6-gliedrigen gesättigten Heterocyclus, der bis zu 2-fach durch $(C_1-C_4)$Alkyl substituiert sein kann,
$R^{12}$ unabhängig voneinander Wasserstoff, $(C_1-C_4)$Alkyl, $(C_1-C_4)$Alkoxy-Carbonyl, Phenyl oder Benzyl, die beide bis zu 3-fach durch $(C_1-C_4)$Alkoxycarbonyl, $(C_1-C_4)$Alkyl, $(C_1-C_4)$Alkoxy oder Halogen substituiert sein können,
$R^{13}$ Wasserstoff, $(C_1-C_4)$Alkyl, Phenyl oder Benzyl, die beide bis zu 3-fach durch Halogen, $(C_1-C_4)$Alkyl, $(C_1-C_4)$Alkoxy, $CF_3$ oder $NO_2$ substituiert sein können, einen Rest der Formeln $-CO-R^{15}$, $-CO-NHR^{15}$,
$-CO-ZR^{15}$, $-S(O)_nR^{15}$ oder $\overset{Z}{\overset{\|}{P}}(ZR^{15})_2$,
$R^{15}$ unabhängig voneinander Wasserstoff, $(C_1-C_4)$Alkyl, Phenyl oder Benzyl, die beide bis zu 3-fach durch Halogen, $(C_1-C_4)$Alkyl, $(C_1-C_4)$Alkoxy, $CF_3$ oder $NO_2$ substituiert sein können, und
m,n = 0, 1, 2, 3 bedeuten,
mit der Maßgabe, daß wenn X = eine direkte Bindung, m = 0, $R^4$ = $(C_1-C_4)$Alkoxycarbonyl, $R^5$ = H und Y = $CH_2$ bedeutet, $R^3$ nicht $(C_1-C_4)$Alkoxycarbonyl und $(C_1-C_4)$Alkoxy$(C_1-C_4)$-alkoxycarbonyl sein darf, enthalten.

Die obengenannte Formel I umfaßt auch alle optischen Isomeren und Isomerengemische der entsprechenden Verbindungen.

Für den Fall, daß $R^4$ = -COZH bedeutet können die Verbindungen der Formel I als Salze vorliegen. Die Erfindung umfaßt alle für die Landwirtschaft einsetzbaren Salze. Als solche kommen beispielsweise infrage die Alkali-oder Erdalkali-salze wie die $Na^+$ $K^+$, $Ca^{2+}$ oder $Mg^{2+}$-Salze oder die Salze mit einem unsubstituierten oder ein-bis vierfach durch organische Reste substituiertem Ammonium.

Hierzu zählen beispielsweise die Ammoniumionen der folgenden Formel

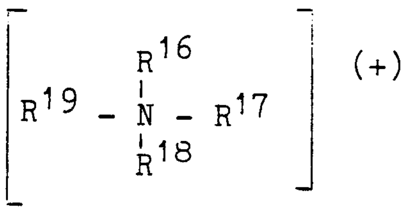

worin
$R^{16}$, $R^{17}$, $R^{18}$ und $R^{19}$ unabhängig voneinander Wasserstoff, $(C_1-C_4)$-Alkyl, das ein bis dreifach durch Halogen, Hydroxy oder $(C_1-C_4)$Alkoxy substituiert sein kann; oder Benzyl, das ein-bis dreifach im Phenylring durch Halogen, $(C_1-C_4)$Alkyl, $(C_1-C_4)$Alkoxy, $CF_3$, $NO_2$ oder CN substituiert sein kann, bedeuten.

Als Heterocyclen für den Rest $-NR^7R^8$ kommen bevorzugt infrage der Morpholin-, Dimethylmorpholin-, Piperidin-oder Pyrrolidin-Ring.

Bevorzugt unter den Verbindungen der Formel I sind solche, bei denen bedeuten: X = 0 oder S, $R^1$ = H oder Fluor, Y = $-C(R^{12})_2-$, O oder S; $R^5$ = H oder $(C_1-C_4)$Alkyl, $R^6$ = $(C_1-C_4)$Alkyl, $(C_1-C_4)$Alkoxycarbonyl oder $(C_1-C_4)$-Alkoxycarbonyl$(C_1-C_4)$alkoxycarbonyl und m = O oder 1 und für den Fall, daß $R^4$ = $CONR^7R^8$ bedeutet, $R^7,R^8$ = H, $(C_1-C_4)$Alkyl oder zusammen einen unsubstituierten oder bis zu vierfach durch $(C_1-C_4)$-Alkyl substituierten Morpholin-, Piperidin-oder Pyrrolidin-Ring bedeutet, und für den Fall, daß $R^4$ = $CO-NR^7NR^9R^{10}$ bedeutet, $R^7$ = H, $R^9$ und $R^{10}$ = H oder zusammen einen Rest $=C(R^{15})_2$ bedeutet. Die übrigen Reste besitzen jeweils die obengenannten Bedeutungen.

Gegenstand der vorliegenden Erfindung sind ferner auch die Verbindungen der Formel I, sowie Salze der Formel I, die neu sind. Es sind dies die Verbindungen der Formel I, mit Ausnahme der Verbindungen, in denen $R^1$ = F, $R^2$ = Cl oder Br, X = Sauerstoff, Y = $CH_2$, S oder $SO_2$, $R^3$ = $(C_1-C_4)$Alkyl, Allyl oder Propargyl und $R^4$ = $-COOR^{3'}$, mit $R^{3'}$ = Alkyl oder ein Alkalimetallatom, $R^5$ = H und m = O bedeuten sowie der Verbindungen, bei denen X = eine direkte Bindung, m = O; $R^1,R^2$ = die obengenannte Bedeutung, $R^3$ = $(C_1-C_4)$Alkoxycarbonyl oder $(C_1-C_4)$Alkoxy$(C_1-C_4)$alkoxycarbonyl, $R^4$ = $(C_1-C_4)$Alkoxy-carbonyl, $R^5$ = H und Y = $CH_2$ bedeuten.

Ein weiterer Gegenstand der Erfindung sind Verfahren zur Herstellung der Verbindungen der allgemeinen Formel I, insbesondere der neuen Verbindungen der Formel I, und ihrer Salze. Die Verfahren sind dadurch gekennzeichnet, daß man

a) ein Isocyanat der Formel II

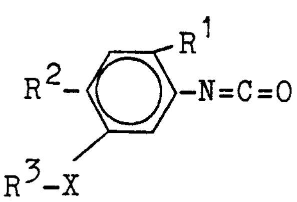

(II)

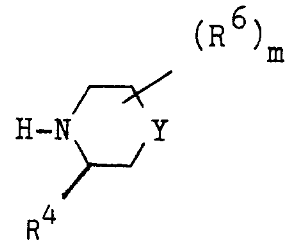

(III)

mit einer Verbindung der Formel III gegebenenfalls in Gegenwart einer Base, oder

b) eine Verbindung der Formel IV mit einer Verbindung der Formel V

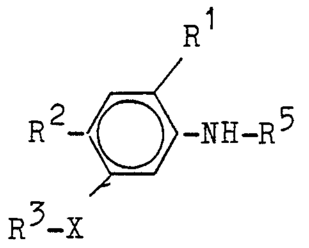

(IV)

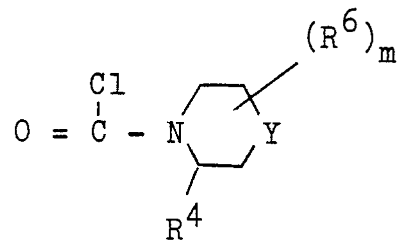

(V)

oder

c) eine Verbindung der Formel VI

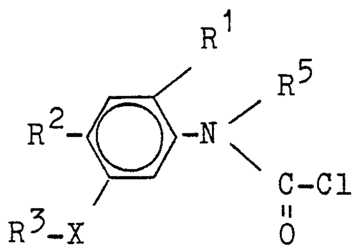

(VI)

mit einer Verbindung der Formel III in Gegenwart einer Base umsetzt und die unter a) bis c) erhaltenen Verbindungen der Formel I im Falle $R^4$ = -CO-ZR gegebenenfalls in ihre Salze überführt.

Bei der Verfahrensvariante a) erfolgt die Umsetzung im Falle $R^4$ = -CO-Z$R^3$ und $R^3$ = H in Gegenwart von insbesondere äquimolaren Mengen einer organischen Base, z.B. eines organischen Amins wie Triethylamin oder Pyridin, oder einer anorganischen Base, z.B. einem Alkali-oder Erdalkalihydroxid, in einem inerten Lösungsmittel, vorzugsweise Wasser, bei Temperaturen zwischen 0°C und 80°C, vorzugsweise zwischen 0 und 40°C.

Die übrigen Verbindungen der Formel I werden bei der Variante a) ohne Basenzusatz in einem unter den Reaktionsbedingungen inerten organischen Lösungsmittel, beispielsweise in Toluol, Aceton, Acetonitril, Dimethylformamid, Ether oder Tetrahydrofuran erhalten. Die Umsetzung erfolgt bei Temperaturen zwischen 0 und 80°C, vorzugsweise bei 0 bis 30°C.

Bei der Verfahrensvariante b) und c) werden als Basen vorzugsweise organische Amine wie Triethylamin oder Pyridin eingesetzt.

Die Verbindungen der Formel V und VI lassen sich auf übliche dem Fachmann geläufige Weise durch Reaktion der Verbindungen der Formel III bzw. IV mit Phosgen gewinnen.

Die Salze der Verbindungen der Formel I mit $R^4$ = -CO-ZH erhält man in bekannter Weise beispielsweise durch Umsetzung der Verbindungen der Formel I mit $R^4$ = -CO-ZH mit einem Metallhydroxid oder einem Amin. Die Umsetzung erfolgt dabei vorzugsweise in einem Lösungsmittel wie z.B. Wasser oder einem niederen aliphatischen Alkohol. Für den Fall, daß die Umsetzung mit einem Amin erfolgt, wird auch das Arbeiten in einem organischen Lösungsmittel, wie z.B. Toluol, Acetonitril oder auch THF, bevorzugt. Die Temperatur für die Umsetzung liegt bei 0-80°C, vorzugsweise bei 0-40°C.

Die erfindungsgemäßen verbindungen der Formel I weisen eine ausgezeichnete herbizide Wirksamkeit gegen ein Breites Spektrum wirtschaftlich wichtiger mono-und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei ist es gleichgültig, ob die Substanzen im Vorsaat-, Vorauflauf-oder Nachauflaufverfahren ausgebracht werden.

Werden die erfindungsgemäßen Verbindungen vor dem Keimen auf die Erdoberfläche appliziert, so wird entweder das Auflaufen der Unkrautkeimlinge vollständig verhindert, oder die Unkräuter wachsen bis zum Keimblattstadium heran, stellen jedoch dann ihr Wachstum ein und sterben schließlich nach Ablauf von drei bis fünf Wochen vollkommen ab.

Bei Applikation der Wirkstoffe auf die grünen Pflanzenteile im Nachauflaufverfahren tritt ebenfalls sehr rasch nach der Behandlung ein drastischer Wachstumsstop ein, und die Unkrautpflanzen bleiben in dem zum Applikations-zeitpunkt vorhandenen Wachstumsstadium stehen oder sterben nach einer gewissen Zeit mehr oder weniger schnell ganz ab, so daß auf diese Weise eine für die Kulturpflanzen schädliche Unkrautkonkurrenz sehr früh und nachhaltig durch den Einsatz der neuen erfindungsgemäßen Mittel beseitigt werden kann.

Obgleich die erfindungsgemäßen Verbindungen eine ausgezeichnete herbizide Aktivität gegenüber mono-und dikotylen Unkräutern aufweisen, werden Kulturpflanzen wirtschaftlich bedeutender Kulturen wie z. B. Weizen, Gerste, Roggen, Reis, Mais, Zuckerrübe, Baumwolle und Soja nur unwesentlich oder gar nicht geschädigt. Die vorliegenden Verbindungen eignen sich aus diesen Gründen sehr gut zur selektiven Bekämpfung von unerwünschtem Pflanzenwuchs in landwirtschaftlichen Nutzpflanzungen.

Darüber hinaus weisen die erfindungsgemäßen Verbindungen wachstumsregulatorische Eigenschaften bei Kulturpflanzen auf. Sie greifen regulierend in den pflanzeneigenen Stoffwechsel ein und können damit zur Ernteerleichterung wie z. B. durch Auslösen von Desikkation, Abszission und Wuchsstauchung eingesetzt werden. Desweiteren eignen sie sich auch zur generellen Steuerung und Hemmung von unerwünschtem vegetativen Wachstums, ohne dabei die Pflanzen abzutöten. Eine Hemmung des vegetativen Wachstums spielt bei vielen mono-und dikotylen Kulturen eine große Rolle, da das Lagern hierdurch verringert oder völlig verhindert werden kann.

Die erfindungsgemäßen Mittel können als Spritzpulver, emulgierbare Konzentrate, versprühbare Lösungen, Stäube mittel, Beizmittel, Dispersionen, Granulate oder Mikrogranulate in den üblichen Zubereitungen angewendet werden.

Spritzpulver sind in Wasser gleichmäßig dispergierbare Präparate, die neben dem Wirkstoff außer gegebenenfalls einem Verdünnungs-oder Inertstoff noch Netzmittel, z. B. polyoxethylierte Alkylphenole, polyoxethylierte Fettalkohole, Alkyl-oder Alkylphenylsulfonate und Dispergiermittel, z. B. ligninsulfonsaures Natrium, 2,2'-di-naphthylmethan-6,6'-disulfonsaures Natrium, dibutylnaphthalinsulfonsaures Natrium oder auch oleoylmethyltaurinsaures Natrium enthalten. Die Herstellung erfolgt in üblicher Weise, z. B. durch Mahlen und Vermischen der Komponenten.

Emulgierbare Konzentrate können z. B. durch Auflösen des Wirkstoffes in einem inerten organischen Lösungsmittel, z. B. Butanol, Cyclohexanon, Dimethylformamid, Xylol oder auch höhersiedenden Aromaten oder Kohlenwasserstoffen unter Zusatz von einem oder mehreren Emulgatoren hergestellt werden. Bei flüssigen Wirkstoffen kann der Lösungsmittelanteil auch ganz oder teilweise entfallen. Als Emulgatoren können beispielsweise verwendet werden Alkylarysulfonsaure Calciumsalze wie Ca-dodecylbenzolsulfonat oder nichtionische Emulgatoren wie Fettsäurepolyglykolester, Alkyl-arylpolyglykolether, Fettalkohol-polyglykolether, Propylenoxid-Ethylenoxid-Kondensations-produkte, Fettalkohol-Propylenoxid-Ethylenoxid-Kondensationsprodukte, Alkylpolyglykolether, Sorbitanfettsäureester, Polyoxethylensorbitanfettsäureester oder Polyoxethylensorbitester.

Stäubemittel kann man durch Vermahlen des Wirkstoffes mit feinverteilten, festen Stoffen z. B. Talkum, natürlichen Tonen wie Kaolin, Bentonit, Pyrophillit oder Diatomeenerde erhalten.

Granulate können entweder durch Verdüsen des Wirkstoffes auf adsorptionsfähiges, granuliertes Inertmaterial hergestellt werden oder durch Aufbringen von Wirkstoffkonzentrationen mittels Bindemitteln, z. B. Polyvinyl-alkohol, polyacrylsaurem Natrium oder auch Mineralölen auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise, gewünschtenfalls in Mischung mit Düngemitteln, granuliert werden.

In Spritzpulvernbeträgt die Wirkstoffkonzentration z. B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 5 bis 80 Gew.-% betragen. Staubförmige Formulierungen enthalten meistens 5 bis 20 Gew.-% an Wirkstoff, versprühbare Lösungen etwa 2 bis 20 Gew.-%. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Daneben enthalten die genannten Wirkstofformulierungen gegebenenfalls die jeweils üblichen Haft-, Netz-, Dispergier-, Emulgier-, Penetrations-, Lösungsmittel, Fülloder Trägerstoffe.

Zur Anwendung werden die in handelsüblicher Form vorliegenden Konzentrate gegebenenfalls in üblicher Weise verdünnt, z. B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Mit den äußeren Bedingungen wie Temperatur, Feuchtigkeit u. a. variiert die erforderliche Aufwandmenge. Sie kann innerhalb weiter Grenzen schwanken, z. B. zwischen 0,005 und 10,0 kg/ha oder mehr Aktivsubstanz, vorzugsweise liegt sie jedoch zwischen 0,01 und 5 kg/ha.

Auch Mischungen oder Mischformulierungen mit anderen Wirkstoffen, wie z. B. Insektiziden, Akariziden, Herbiziden, Düngemitteln, Wachstumsregulatoren oder Fungiziden sind gegebenenfalls möglich.

Nachstehend seien einige Formulierungsbeispiele aufgeführt:

Ein Stäubemittel wird erhalten, indem man 10 Gewichtsteile Wirkstoff und 90 Gewichtsteile Talkum oder Inertstoff mischt und in einer Schlagmühle zerkleinert.

Ein in Wasser leicht dispergierbares, benetzbares Pulver wird erhalten, indem man 25 Gewichtsteile Wirkstoff, 64 Gewichtsteile kaolinhaltigen Quarz als Inertstoff, 10 Gewichtsteile ligninsulfonsaures Kalium und 1 Gewichtsteil oleoylmethyltaurinsaures Natrium als Netz-und Dispergiermittel mischt und in einer Stiftmühle mahlt.

Ein in Wasser leicht dispergierbares Dispersionkonzentrat wird erhalten, indem man 20 Gewichtsteile Wirkstoff mit 6 Gewichtsteilen Alkylphenolpolyglykolether ($^{(R)}$Triton X 207), 3 Gewichtsteilen Isotridecanolpolyglykolether (8 AeO) und 71 Gewichtsteilen paraffinischem Mineralöl (Siedebereich z. B. ca. 255 bis über 377 °C) mischt und in einer Reibkugelmühle auf eine Feinheit von unter 5 Mikron vermahlt.

Ein emulgierbares Konzentrat wird erhalten aus 15 Gewichts teilen Wirkstoff, 75 Gewichtsteilen Cyclohexanon als Lösungsmittel und 10 Gewichtsteilen oxethyliertes Nonyl-phenol (10 AeO) als Emulgator.

## Chemische Beispiele

### Beispiel 1

1-(4-Chlor-2-fluor-5-methoxy-phenyl-carbamoyl)-piperidin-2-carbonsäure

Es wurden 12,9 g (0,1 mol) 2-Piperidincarbonsäure und 4,0 g (0,1 mol) Natriumhydroxid in 300 ml Wasser gelöst. Bei 20 °C wurde 20,2 g (0,1mol) 4-Chlor-2-fluor-5-methoxy-phenylisocyanat, gelöst in 30 ml Aceton, zugetropft und 2 h bei 20 °C nachgerührt. Mit konz. Salzsäure wurde auf pH 1 angesäuert und der Niederschlag abgesaugt. Dann wurde säurefrei gewaschen und bei 60 °C getrocknet. Es wurden 29,8 g (90 % d. Th.) 1-(4-Chlor-2-fluor-5-methoxy-phenyl-carbamoyl)-piperidin-2-carbonsäure in Form farbloser Kristalle mit Schmp. 158 -160 °C erhalten.

### Beispiel 2

1-(4-Chlor-2-fluor-5-methoxy-phenylcarbamoyl)-piperidin-2-carbonsäureethylester

Es wurden 15,7 g (0,1 mol) 2-Piperidincarbonsäureethyl-ester in 200 ml Toluol gelöst und bei 20 °C 20,2 g (0,1 mol) 4-Chlor-2-fluor-5-methoxy-phenylisocyanat gelöst in 50 ml Toluol, zugetropft. Es wurde 2 h bei 20 °C gerührt und das Toluol unter vermindertem Druck abdestilliert. Man erhielt 35,9 g (100 % d. Th.) 1-(4-Chlor-2-fluor-5-methoxy-phenyl-carbamoyl)-piperidin-2-carbonsäure-ethylester in Form eines hellgelben Öles, das nach mehreren Tagen kristallisierte. Schmp. 122 - 126°C.

### Beispiel 3

1-(4-Chlor-2-fluor-5-methoxy-phenylcarbamoyl)-piperidin-2-carbonsäure-Kaliumsalz

Es wurden 15,5 g (0,05 mol) 1-(4-Chlor-2-fluor-5-methoxy-phenylcarbamoyl)-piperidin-2-carbonsäure - (Bsp. 1) in 200 ml Methanol gelöst und unter Eiskühlung 3,3 g Kaliumhydroxid 85%ig, gelöst in 50 ml Methanol, zugetropft. Es wurde 30 min bei 0 °C gerührt und das Methanol und das gebildete Wasser abdestilliert. Der Rückstand wurde bei 60 °C getrocknet. Man erhielt 18,4 g (100 % d. Th.) 1-(4-Chlor-2-fluor-5-methoxy-phenylcarbamoyl)-piperidin-2-carbon-säure-Kaliumsalz in Form farbloser Kristalle mit Schmp. 118 -120°C.

Analog wurden die Verbindungen der Tabelle 1 hergestellt.

Tabelle 1A ; Verbindungen der Formel I mit $Y = CH_2$, $R^5$=H, m=O

| Bsp. | $R^1$ | $R^2$ | $R^3$ | $R^4$ | X | Fp[°C] |
|---|---|---|---|---|---|---|
| 4 | F | Cl | $CH(CH_3)_2$ | $CO_2H$ | O | |
| 5 | F | Cl | " | $CO_2C_2H_5$ | O | |
| 6 | F | Cl | " | $CO_2^{\ominus}$ $NH_4^{\oplus}$ | O | |
| 7 | F | Cl | $CH_3$ | $CO_2^{\ominus\oplus}NH_3CH_2CH_2OH$ | O | |
| 8 | F | Cl | $CH_3$ | $CO$ $NH_2$ | O | |
| 9 | H | Cl | $CH_3$ | $CO_2H$ | O | |
| 10 | H | Cl | $CH_3$ | $CO_2C_2H_5$ | O | |
| 11 | F | Cl | $CH_2-CO_2C_2H_5$ | $CO_2H$ | O | |
| 12 | F | Cl | $CH_2-CO_2C_2H_5$ | $CO_2C_2H_5$ | O | |
| 13 | F | Cl | $CH_2-CO_2C_2H_5$ | $CO_2^{\ominus}$ $K^{\oplus}$ | O | |
| 14 | F | Cl | $CH(CH_3)CO_2C_2H_5$ | $CO_2H$ | O | |
| 15 | F | Cl | " | $CO_2C_2H_5$ | O | |
| 16 | H | Cl | " | " | O | |
| 17 | H | Cl | " | $CO_2H$ | O | |

Fortsetzung Tabelle 1A

| Bsp. | $R^1$ | $R^2$ | $R^3$ | $R^4$ | X | Fp [°C] |
|---|---|---|---|---|---|---|
| 18 | F | Cl | $CH(CH_3)CO_2C_5H_{11}$ | $CO_2C_2H_5$ | O | |
| 19 | F | Cl | $CH_2-CO_2C_5H_{11}$ | $CO_2H$ | O | |
| 20 | F | Cl | $CH_3$ | CN | O | |
| 21 | F | Cl | $CH(CH_3)_2$ | 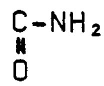 | O | |
| 22 | F | Cl | " | 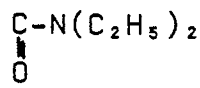 | O | |
| 23 | F | Cl | $CH_3$ | 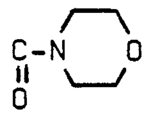 | O | |
| 24 | F | Cl | $CH_3$ | $CO_2^-$ $^+N(CH_3)_4$ | O | |
| 25 | F | Cl | $CH(CH_3)_2$ | $CO_2C_2H_5$ | O | |
| 26 | F | Cl | $CH_3$ | $CO_2^{\ominus}$ $Na^+$ | O | |
| 27 | F | Cl | $CH_3$ | $CO_2^{\ominus}$ $NH_4^{\oplus}$ | O | |
| 28 | F | Cl | $CH_3$ | 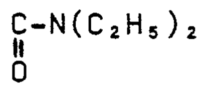 | O | |
| 29 | H | Cl | $CH_3$ | $CO_2CH_3$ | O | |
| 30 | F | Cl | $CH_3$ | $CO_2H$ | S | |
| 31 | F | Cl | $CH(CH_3)_2$ | $CO_2C_2H_5$ | S | |
| 32 | F | Cl | $CH_3$ | $CO_2^{\ominus}$ $K^{\oplus}$ | S | |
| 33 | F | Cl | $CH_3$ | 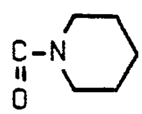 | S | |
| 34 | H | Cl | $CH(CH_3)_2$ | $CO_2H$ | S | |
| 35 | F | Cl | $CH_2CO_2CH_3$ | $CO_2C_2H_5$ | S | |
| 36 | F | Cl | $CH(CH_3)CO_2C_2H_5$ | $CO_2H$ | S | |
| 37 | F | Cl | $CH(CH_3)CO_2C_5H_{11}$ | $CO_2C_2H_5$ | S | |
| 38 | F | Cl | $CH_2CO_2C_4H_9$ | $CO_2H$ | S | |
| 39 | F | Cl | $CH_3$ | $CO_2^{\ominus}$ $K^{\oplus}$ | S | |
| 40 | F | Cl | $CH(CH_3)_2$ | $CO_2^{\ominus}$ $NH_4^{\oplus}$ | S | |

...

Fortsetzung Tabelle 1A

| Bsp. | $R^1$ | $R^2$ | $R^3$ | $R^4$ | X | Fp [°C] |
|---|---|---|---|---|---|---|
| 41 | H | Cl | $CH_3$ | $CO_2H$ | S | |
| 42 | H | Cl | $CH(CH_3)_2$ | $CO_2C_2H_5$ | S | |
| 43 | H | Cl | $CH(CH_3)CO_2C_2H_5$ | $CO_2C_2H_5$ | S | |
| 44 | H | Cl | $CH_2CO_2C_6H_{11}$ | $CO_2H$ | S | |
| 45 | H | Cl | $CH_2CO_2C_2H_5$ | $CON(C_2H_5)_2$ | S | |
| 46 | H | Cl | " | $CO_2^{\ominus}$ $NH_4^{\oplus}$ | S | |
| 47 | H | Cl | H | $CONH-C_6H_4-4-Cl$ | - | 163-166 |
| 48 | H | Cl | H | $CONH-C_6H_3-2,6-(C_2H_5)_2$ | - | 222-224 |
| 49 | F | Cl | $CH_3$ | " | O | 198-200 |
| 50 | F | Cl | $CH_3$ | COON 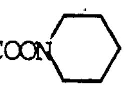 | O | 50-62 |
| 51 | F | Cl | $CH_3$ | $CONH-N=CH-C_6H_5$ | O | gelbes Harz |
| 52 | H | Cl | H | " | - | 148-151 |
| 53 | H | Cl | H | $CONH-C_6H_{11}$ (Cyclo) | - | 105 (Z) |
| 54 | F | Cl | $CH_3$ | " | O | 102 (Z) |
| 55 | H | Cl | H | CN | - | 137-141 |
| 56 | F | Br | $CH_3$ | CN | O | 126-130 |
| 57 | H | Cl | H | $CONH-O-CH_2-C_6H_5$ | - | gelbes Harz |
| 58 | F | Cl | $CH_3$ | " | O | " |
| 59 | F | Cl | $CH_3$ | $CON(CH_3)C_6H_5$ | O | 154-158 |
| 60 | H | $HC(CH_3)_2$ | H | CN | - | 122-125 |
| 61 | F | Cl | $CH_3$ | $-C(=N-OH)NH_2$ | O | 75 |
| 62 | H | $Cl_2HC-CF_2-O-$ | $CH_3$ | CN | - | gelbes Harz |
| 63 | H | $CH_3$ | $CH_3$ | CN | O | 140 |
| 64 | H | Cl | H | $CON(C_2H_5)C_6H_5$ | - | 145-148 |
| 65 | F | Cl | $CH_3$ | " | - | 140-144 |
| 66 | H | Cl | H | $-CONH-NH-CO_2C_2H_5$ | - | 57-61 |
| 67 | F | Cl | $CH_3$ | " | O | gelbes Harz |
| 68 | H | Cl | H | $-C(=N-OH)NH_2$ | - | 61-66 |
| 69 | H | Cl | $CO_2C_2H_5$ | CN | - | |
| 70 | H | Br | $CO_2C_2H_5$ | CN | - | |

Fortsetzung Tabelle 1A

| Bsp. | $R^1$ | $R^2$ | $R^3$ | $R^4$ | X | Fp /°C/ |
|---|---|---|---|---|---|---|
| 71 | H | Cl | $CO_2\overset{CH_3}{\overset{\vert}{C}}H-CO_2C_2H_5$ | $CO_2C_2H_5$ | - | |
| 72 | H | Cl | $CO_2\overset{CH_3}{\overset{\vert}{C}}H-CO_2CH_2CF_3$ | $CO_2C_2H_5$ | - | |
| 73 | F | Cl | $CO_2\overset{CH_3}{\overset{\vert}{C}}H-CO_2C_2H_5$ | $CO_2C_2H_5$ | - | |
| 74 | F | Cl | $CO_2C_2H_5$ | CN | | |
| 75 | F | Cl | $CO_2C_2H_5$ | $CONH_2$ | - | |
| 76 | H | Br | $CO_2\overset{CH_3}{\overset{\vert}{C}}H-CO_2C_2H_5$ | $CONH-N=CH-C_6H_5$ | - | |
| 77 | H | Br | $CO_2\overset{CH_3}{\overset{\vert}{C}}HCO_2C_2H_5$ | CO-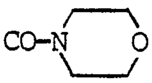 | - | |
| 78 | H | Cl | $CO_2C_2H_5$ | CO-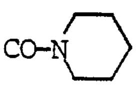 | - | |
| 79 | H | Cl | $CO_2C_2H_5$ | $CON(CH_3)C_6H_5$ | - | |
| 80 | H | Br | $CO_2C_2H_5$ | $CONHNH_2$ | - | |

Beispiel 81

4-(4-Chlor-2-fluor-5-methoxy-phenylcarbamoyl)-1,4-thiazan-3-carbonsäureethylester

3,5 g (0,02 mol) 1,4-Thiazan-3-carbonsäureethylester wurden in 40 ml Diethylether gelöst und bei 20°C 4,0 g (0,02 mol) 4-Chlor-2-fluor-5-methoxy-phenylisocyanat, gelöst in 20 ml Diethylether, zugetropft. Man rührte 2 Stunden bei 20°C. Anschließend wurde der Diethylether unter vermindertem Druck abdestilliert.

Man erhielt 7,5 g (100 % d.Th.) 4-(4-Chlor-2-fluor-5-methoxy-phenylcarbamoyl)-1,4-thiazan-3-carbonsäureethylester in Form eines hellgelben zähen Öles.

Analog der in den Beispielen 1, 2 oder 81 beschriebenen Verfahrensweisen lassen sich die Verbindungen der folgenden Tabellen 1B bis 1E herstellen.

Tabelle 1B ; Verbindungen der Formel I mit Y = S, SO oder $SO_2$
$R^5$= H; m= O;

| Bsp. | $R^1$ | $R^2$ | $R^3$ | $R^4$ | X | Y | Fp(°C) |
|---|---|---|---|---|---|---|---|
| 82 | F | Cl | $CH_3$ | $CO_2C_4H_9$ | O | S | gelbes Öl |
| 83 | F | Cl | $CH_3$ | $CO_2H$ | O | S | |
| 84 | F | Cl | $CH(CH_3)_2$ | $CO_2C_2H_5$ | O | S | |
| 85 | F | Cl | $CH(CH_3)CO_2C_2H_5$ | $CO_2C_2H_5$ | O | S | |
| 86 | F | Cl | $CH(CH_3)CO_2C_2H_5$ | $CO_2H$ | O | S | |
| 87 | F | Cl | $CH_3$ | $CO_2^{\ominus}K^{\oplus}$ | O | S | |
| 88 | F | Cl | $CH_3$ | CN | O | S | |
| 89 | F | Cl | $CH_3$ | $C(=O)-N(C_2H_5)_2$ | O | S | ... |
| 90 | F | Cl | $CH_3$ | $CO_2C_2H_5$ | S | S | |
| 91 | F | Cl | $CH(CH_3)CO_2C_2H_5$ | $CO_2C_2H_5$ | S | S | |
| 92 | F | Cl | $CH(CH_3)CO_2C_2H_5$ | $CO_2H$ | S | S | |
| 93 | F | Cl | $CH_3$ | $CO_2C_2H_5$ | O | SO | |
| 94 | F | Cl | $CH_3$ | $CO_2H$ | O | SO | |
| 95 | F | Cl | $CH_3$ | $CO_2C_2H_5$ | O | $SO_2$ | |
| 96 | F | Cl | $CH_3$ | $CO_2H$ | O | $SO_2$ | |
| 97 | H | Cl | H | $CO_2C_4H_9$ | - | S | 118-120 |
| 98 | H | Cl | H | CN | - | S | 180 |
| 99 | H | Cl | H | $CO_2C_2H_5$ | - | S | 157-153 |
| 100 | F | Cl | $CH_3$ | $CONH_2$ | - | S | 136-141 |
| 101 | F | Cl | $CH_3$ | $CONHN=CH-C_6H_5$ | - | S | gelbes Harz |
| 102 | F | Cl | $CH_3$ | $CON(C_2H_5)C_6H_5$ | - | S | 149-152 |
| 103 | H | Cl | $CO_2CH(CH_3)CO_2CH_3$ | $CO_2C_2H_5$ | - | S | |
| 104 | H | Cl | " | CN | - | S | |

Tabelle 1C; Verbindungen mit Y= O, $-NR^{13}$ oder $-C(R^{12})_2-$ ;
m= O, $R^5$ = H

| Bsp. | $R^1$ | $R^2$ | $R^3$ | $R^4$ | X | Y | Fp °C |
|---|---|---|---|---|---|---|---|
| 105 | H | Cl | H | CN | - | O | 103-108 |
| 106 | F | Cl | $CH_3$ | CN | O | O | 71-75 |
| 107 | H | Cl | H | $CO_2H$ | - | O | 95-98 |
| 108 | F | Cl | $CH_3$ | $CO_2C_2H_5$ | O | O | gelbes Öl |
| 109 | F | Cl | $CH_3$ | $CONH_2$ | O | O | |
| 110 | F | Cl | $CH_3$ | $CO_2H$ | O | O | glasartig |
| 111 | F | Cl | $CH_3$ | $CONH-N=CHC_6H_5$ | O | O | |
| 112 | H | Br | $CO_2C_2H_5$ | CN | - | O | |
| 113 | H | Cl | $CO_2C_2H_5$ | $CO_2C_2H_5$ | - | O | |
| 114 | H | Br | $CO_2\underset{}{\overset{CH_3}{\overset{\vert}{C}}}HCO_2C_2H_5$ | CN | - | O | gelbes Harz |
| 115 | H | Br | " | $CO_2C_2H_5$ | - | O | " |
| 116 | H | Cl | " | $CONH_2$ | - | O | |
| 117 | H | Cl | $CO_2C_2H_5$ | $CONH-N=CH-C_6H_5$ | - | O | |
| 118 | H | Cl | H | CN | - | $NCH_2C_6H_5$ | gelbes Harz |
| 119 | F | Cl | $CH_3$ | CN | O | " | |
| 120 | F | Cl | $CH_3$ | $CO_2C_2H_5$ | O | " | " |
| 121 | F | Cl | $CH_3$ | CN | O | $NCH_3$ | |
| 122 | F | Cl | $CH_3$ | $CO_2C_2H_5$ | O | $NCH_3$ | |
| 123 | F | Cl | $CH_3$ | $CO_2C_2H_5$ | O | $N-C_6H_5$ | |
| 124 | F | Cl | $CH_3$ | $CO_2C_2H_5$ | O | $N-\underset{O}{\underset{\|}{C}}-CH_3$ | |
| 125 | F | Cl | $CH_3$ | CN | O | $N-\underset{O}{\underset{\|}{C}}-NHCH_3$ | |
| 126 | F | Cl | $CH_3$ | $CO_2C_2H_5$ | O | $N\overset{O}{\overset{\|}{P}}(OC_2H_5)_2$ | |
| 127 | F | Cl | $CH_3$ | CN | O | $NSO_2C_6H_4-4-CH_3$ | |
| 128 | H | Cl | $CO_2C_2H_5$ | CN | - | $NCH_2C_6H_5$ | |
| 129 | H | Br | $CO_2\overset{CH_3}{\overset{\vert}{C}}HCO_2C_2H_5$ | $CO_2C_2H_5$ | - | " | |
| 130 | H | Br | $CO_2C_2H_5$ | CN | - | $N-\underset{O}{\underset{\|}{C}}-OCH_3$ | |
| 131 | H | Cl | $CO_2\overset{CH_3}{\overset{\vert}{C}}HCO_2C_2H_5$ | $CO_2C_2H_5$ | - | $NCH_3$ | |
| 132 | H | Cl | $CO_2C_2H_5$ | $CO_2C_2H_5$ | - | $NC_6H_5$ | |

| Bsp. | $R^1$ | $R^2$ | $R^3$ | $R^4$ | X | Y | Fp °C |
|---|---|---|---|---|---|---|---|
| 133 | H | Cl | H | CN | - | $CH(CH_3)$ | 125-130 |
| 134 | F | Cl | $CH_3$ | CN | O | $CH(CH_3)$ | gelbes Öl |
| 135 | H | Cl | $CO_2CH(CH_3)CO_2C_2H_5$ | CN | - | $CH(CH_3)$ | gelbes Öl |
| 136 | F | Cl | $CH_3$ | $CO_2C_2H_5$ | O | $CH(CH_3)$ | " |
| 137 | F | Cl | $CH_3$ | $CONH_2$ | O | $CH(CH_3)$ | |
| 138 | F | Cl | $CH_3$ | $CO_2C_2H_5$ | O | $C(CH_3)_2$ | |
| 139 | F | Cl | $CH_3$ | CN | O | $CH-C_6H_5$ | |
| 140 | H | Br | $CO_2C_2H_5$ | CN | - | $CH(CH_3)$ | |
| 141 | H | Cl | $CO_2CH(CH_3)CO_2C_2H_5$ | $CO_2C_2H_5$ | - | $CH(CH_3)$ | |
| 142 | F | Cl | $CH_3$ | $CO_2C_2H_5$ | O | $CH(CH_2-C_6H_5)$ | |
| 143 | H | Cl | $CO_2CH_2CF_3$ | $CO_2C_2H_5$ | - | $CH(CH_3)$ | |

Tabelle 1D; Verbindungen mit $R^5$ = H und $m \neq 0$

| Bsp. | $R^1$ | $R^2$ | $R^3$ | $R^4$ | X | Y | $(R^6)_m$ | Fp °C |
|---|---|---|---|---|---|---|---|---|
| 144 | F | Cl | $CH_3$ | 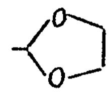 | O | $CH_2$ | $6-CH_3$ | gelbes Harz |
| 145 | H | Cl | H | " | – | $CH_2$ | $6-CH_3$ | " |
| 146 | F | Cl | $CH_3$ | $-CO_2C_2H_5$ | O | $CH_2$ | $6-CH_3$ | Sirup |
| 147 | F | Cl | $CH_3$ | $-CO_2C_2H_5$ | O | $CH_2$ | $6-CO_2C_2H_5$ | oranges Harz |
| 148 | H | Cl | H | $-CO_2C_2H_5$ | – | $CH_2$ | $6-CO_2C_2H_5$ | " |
| 149 | F | Cl | $CH_3$ | $-CO_2\overset{CH_3}{\overset{\mid}{C}}HCO_2C_2H_5$ | O | $CH_2$ | $6-CO_2\overset{CH_3}{\overset{\mid}{C}}HCO_2C_2H_5$ | " |
| 150 | H | Cl | H | " | – | $CH_2$ | " | " |
| 151 | F | Cl | $CH_3$ | $-CO_2CH(CH_3)_2$ | O | $CH_2$ | $6-CO_2CH(CH_3)_2$ | " |
| 152 | F | Cl | $CH_3$ | $-CO_2CH_2CF_3$ | O | $CH_2$ | $6-CO_2CH_2CF_3$ | " |
| 153 | F | Cl | $CH_3$ | $-CO_2C_2H_5$ | O | $CH_2$ | $5-CO_2C_2H_5$ | " |
| 154 | F | Cl | $CH_3$ | $-CO_2C_2H_5$ | O | $CH_2$ | $5-C_2H_5$ | " |
| 155 | F | Cl | $CH_3$ | $-CO_2C_2H_5$ | O | $CH_2$ | $5-CO_2C_2H_5$ | " |
| 156 | F | Cl | $CH_3$ | $-CO_2C_2H_5$ | O | $CH_2$ | $3-CO_2C_2H_5$ | " |
| 157 | F | Cl | $CH_3$ | $-CO_2CH_2CF_3$ | O | $CH_2$ | $3-CO_2CH_2CF_3$ | " |
| 158 | H | Cl | H | $-CO_2CH_2CF_3$ | – | $CH_2$ | $3-CO_2CH_2CF_3$ | " |

Fortsetzung Tabelle 1D:

| Bsp. | $R^1$ | $R^2$ | $R^3$ | $R^4$ | X | Y | $(R^6)_m$ | Fp °C |
|---|---|---|---|---|---|---|---|---|
| 159 | F | Cl | $CH_3$ | $-CO_2C_2H_5$ | O | $CH_2$ | $4-CH_3$ | |
| 160 | F | Cl | $CH_3$ | $-CO_2C_2H_5$ | O | $CH_2$ | $4-CO_2C_2H_5$ | |
| 161 | H | Cl | $CO_2C_2H_5$ | $-CO_2C_2H_5$ | – | $CH_2$ | $6-CO_2C_2H_5$ | |
| 162 | H | Br | $CO_2C_2H_5$ | $-CO_2C_2H_5$ | – | $CH_2$ | $6-CH_3$ | |
| 163 | H | Br | $CO_2\overset{CH_3}{C}HCO_2C_2H_5$ | $-CO_2C_2H_5$ | – | $CH_2$ | $6-CO_2C_2H_5$ | |
| 164 | H | Cl | $CO_2CH_2CF_3$ | $-CO_2C_2H_5$ | – | $CH_2$ | $6-CO_2C_2H_5$ | |
| 165 | H | Cl | $CO_2CH_2CF_3$ | $-CO_2C_2H_5$ | – | $CH_2$ | $3-CO_2C_2H_5$ | |
| 166 | H | Cl | $CO_2CH_2CF_3$ | $-CO_2\overset{CH_3}{C}HCO_2C_2H_5$ | – | $CH_2$ | $6-CO_2\overset{CH_3}{C}HCO_2CH_5$ | |
| 167 | H | Cl | $CO_2C_2H_5$ | $-CO_2\overset{CH_3}{C}HCO_2C_2H_5$ | – | $CH_2$ | $5-C_2H_5$ | |
| 168 | F | Cl | $CH_3$ | $-CO_2C_2H_5$ | O | S | $5-CH_3$ | 76-80 |
| 169 | H | Cl | H | $-CO_2C_2H_5$ | – | S | $5-C_6H_5$ | gelbes Harz |
| 170 | F | Cl | $CH_3$ | $-CO_2C_2H_5$ | O | S | $5-C_6H_5$ | " |
| 171 | H | Cl | H | $-CO_2C_2H_5$ | O | S | $5-CH_3$ | " |
| 172 | F | Cl | $CH_3$ | $-CO_2C_2H_5$ | O | S | $2,2,5-(CH_3)_3$ | " |
| 173 | H | Cl | H | $-CN$ | – | O | $2,6-(CH_3)_2$ | 81-82 |
| 174 | F | Cl | $CH_3$ | $-CN$ | O | O | $2,6-(CH_3)_2$ | 186-188 |

Fortsetzung Tabelle 1D:

| Bsp. | $R^1$ | $R^2$ | $R^3$ | $R^4$ | X | Y | $(R^6)_m$ | Fp °C |
|---|---|---|---|---|---|---|---|---|
| 175 | H | Cl | H | $-CO_2C_2H_5$ | – | S | 5-(2,4$Cl_2$-$C_6H_3$) | Harz |
| 176 | F | Cl | $CH_3$ | $-CO_2C_2H_5$ | O | S | " | " |
| 177 | H | Cl | H | $-CO_2C_2H_5$ | – | S | 2,2-$(CH_3)_2$, 5-$C_6H_5$ | 161-164 |
| 178 | F | Cl | $CH_3$ | $-CO_2C_2H_5$ | O | S | 2,2-$(CH_3)_2$, 5-$C_6H_5$ | Harz |
| 179 | H | Cl | H | $-CO_2C_2H_5$ | – | S | 2,2-$(CH_3)_2$, 5-(2,4$Cl_2$-$C_6H_3$) | Harz |
| 180 | F | Cl | $CH_3$ | $-CO_2C_2H_5$ | O | S | 2,2-$(CH_3)_2$, 5-(2,4$Cl_2$-$C_6H_3$) | Harz |
| 181 | H | Cl | H | $-CO_2H$ | – | O | 2,6-$(CH_3)_2$ | 120-122 |
| 182 | F | Cl | $CH_3$ | $-CO_2H$ | – | O | 2,6-$(CH_3)_2$ | Harz |
| 183 | H | $CH(CH_3)_2$ | H | -CN | – | O | 2,6-$(CH_3)_2$ | Harz |
| 184 | H | $CH(CH_3)_2$ | H | $-CO_2C_2H_5$ | – | S | 5-(2,4$Cl_2$-$C_6H_3$) | Harz |
| 185 | H | Cl | H | -CN | – | $CH_2$ | 3,5-$(CH_3)_2$ | 99-103 |
| 186 | F | Cl | $CH_3$ | -CN | O | $CH_2$ | 3,5-$(CH_3)_2$ | 96-98 |
| 187 | H | $CH(CH_3)_2$ | H | $-CO_2C_2H_5$ | – | S | 2,2-$(CH_3)_2$, 5-$C_6H_5$ | 124-128 |

Fortsetzung Tabelle 1E: Verbindungen mit $R^5 \neq H$ und $m \neq 0$

| Bsp. | $R^1$ | $R^2$ | $R^3$ | $R^4$ | X | Y | $(R^6)_m$ | $R^5$ | Fp °C |
|---|---|---|---|---|---|---|---|---|---|
| 188 | F | Cl | $CH_3$ | $CO_2C_2H_5$ | O | $CH_2$ | - | $CH_3$ | 92-94 |
| 189 | F | Cl | $CH_3$ | $CO_2H$ | O | $CH_2$ | - | $CH_3$ | 143-46 (Z) |
| 190 | H | Cl | H | $CO_2C_2H_5$ | - | $CH_2$ | - | $CH_3$ | gelbes Öl |
| 191 | F | Cl | $CH_3$ | $CO_2C_2H_5$ | O | $CH_2$ | - | $CH_2CO_2CH_3$ | " |
| 192 | F | Cl | $CH_3$ | $CO_2C_2H_5$ | O | $CH_2$ | $6-CO_2C_2H_5$ | $CH_3$ | " |
| 193 | F | Cl | $CH_3$ | $CO_2CH_2F_3$ | O | $CH_2$ | $6-CO_2CH_2CF_3$ | $CH_3$ | " |
| 194 | F | Cl | $CH_3$ | $CO_2CH_2CF_3$ | O | $CH_2$ | $3-CO_2CH_2CF_3$ | $CH_3$ | " |
| 195 | F | Cl | $CH_3$ | $CO_2C_2H_5$ | O | $CH_2$ | $5-CO_2C_2H_5$ | $CH_3$ | |
| 196 | H | Cl | $CO_2C_2H_5$ | $CO_2C_2H_5$ | - | $CH_2$ | - | $CH_3$ | |
| 197 | H | Cl | $CO_2CH(CH_3)CO_2C_2H_5$ | $CO_2C_2H_5$ | - | $CH_2$ | - | $CH_3$ | |
| 198 | H | Br | $CO_2CH(CH_3)CO_2C_2H_5$ | $CO_2C_2H_5$ | - | $CH_2$ | $6-CO_2C_2H_5$ | $CH_3$ | |

Biologische Beispiele

Die Schädigung der Unkrautpflanzen bzw. die Kulturpflanzenverträglichkeit wurde gemäß einem Schlüssel bonitiert, in dem die Wirksamkeit durch Wertzahlen von 0 -5 ausgedrückt ist. Dabei bedeutet:

0 = ohne Wirkung bzw. Schaden
1 = 0 -20 % Wirkung bzw. Schaden
2 = 20 -40 % Wirkung bzw. Schaden
3 = 40 -60 % Wirkung bzw. Schaden
4 = 60 -80 % Wirkung bzw. Schaden
5 = 80 -100 % Wirkung bzw. Schaden

1. Unkrautwirkung im Vorauflauf

Samen bzw. Rhizomstücke von mono-und dikotylen Unkrautpflanzen wurden in Plastiktöpfen (Ø = 9 cm) in sandiger Lehmerde ausgelegt und mit Erde abgedeckt. Die in Form von benetzbaren Pulvern von Emulsionskonzentraten formulierten erfindungsgemäßen Verbindungen wurden dann als wäßrige Suspensionen bzw. Emulsionen mit einer Wasseraufwandmenge von umgerechnet 600 l/ha in unterschiedlichen Dosierungen auf die Oberfläche der Abdeckerde appliziert.
Nach der Behandlung wurden die Töpfe im Gewächshaus aufgestellt und unter guten Wachstumsbedingungen für die Umkrautpflanzen gehalten (Temperatur 23 plus/minus 1 °C, relative Luftfeuchte 60 -80 %).

Die optische Bonitur der Pflanzen bzw. Auflaufschäden erfolgte nach dem Auflaufen der Versuchspflanzen nach einer Versuchszeit von 3 -4 Wochen im Vergleich zu unbehandelten Kontrollen.
Die Ergebnisse sind in der nachfolgenden Tabelle II zusammengefaßt.

Tabelle II

| Bsp.-Nr. | Dosis kg a.i./ha | herbizide Wirkung SIA | CRS | LOM | ECG |
|---|---|---|---|---|---|
| 1 | 2,5 | 5 | 5 | 5 | 5 |
| 2 | 2,5 | 5 | 5 | 5 | 5 |
| 3 | 2,5 | 5 | 5 | 5 | 5 |
| 8 | 2,5 | 5 | 5 | 5 | 5 |
| 20 | 2,5 | 5 | 4 | 5 | 5 |
| 23 | 2,5 | 5 | 5 | 5 | 5 |
| 81 | 2,5 | 5 | 5 | 5 | 5 |
| 82 | 2,5 | 5 | 5 | 5 | 3 |
| 50 | 2,5 | 5 | 5 | 5 | 5 |
| 52 | 2,5 | 4 | 4 | 4 | 4 |
| 54 | 2,5 | 5 | 5 | 5 | 5 |
| 56 | 2,5 | 5 | 5 | 5 | 5 |
| 57 | 2,5 | 3 | 5 | 4 | 3 |
| 58 | 2,5 | 5 | 5 | 5 | 5 |
| 97 | 2,5 | 5 | 5 | 5 | 3 |
| 99 | 2,5 | 5 | 5 | 5 | 4 |
| 106 | 2,5 | 5 | 5 | 5 | 5 |
| 134 | 2,5 | 5 | 5 | 5 | 5 |
| 144 | 2,5 | 5 | 4 | 5 | 3 |
| 146 | 2,5 | 5 | 5 | 5 | 5 |
| 168 | 2,5 | 5 | 5 | 5 | 5 |
| 170 | 2,5 | 4 | 4 | 2 | 2 |
| 174 | 2,5 | 5 | 5 | 5 | 5 |
| 176 | 2,5 | 5 | 5 | 5 | 5 |
| 188 | 2,5 | 5 | 5 | 5 | 4 |
| 189 | 2,5 | 4 | 2 | 3 | 0 |

2. Unkrautwirkung im Nachauflauf

Samen bzw. Rhizomstücke von mono-und dikotylen Unkräutern wurden in Plastiktöpfen (Ø = 9 cm) in sandigem Lehmboden ausgelegt, mit Erde abgedeckt und im Gewächshaus unter guten Wachstumsbedingungen angezogen. Drei Wochen nach Aussaat wurden die Versuchspflanzen im Dreiblattstadium Behandelt.

Die als Spritzpulver bzw. als Emulsionskonzentrate formulierten erfindungsgemäßen Verbindungen wurden in verschiedenen Dosierungen mit einer Wasseraufwandmenge von umgerechnet 600 l/ha auf die grünen Pflanzenteile gesprüht und nach ca. 3 -4 Wochen Standzeit der Versuchspflanzen im Gewächshaus unter

optimalen Wachstumsbedingungen (Temperatur 23 plus/minus 1 °C, relative Luftfeuchte 60 -80 %) die Wirkung der Präparate optisch im Vergleich zu unbehandelten Kontrollen bonitiert.
Die erfindungsgemäßen Verbindungen weisen auch im Nachauflauf eine gute herbizide Wirksamkeit gegen ein breites Spektrum wirtschaftlich wichtiger Ungräser und Unkräuter auf.
Die Ergebnisse sind in der nachfolgenden Tabelle III zusammengefaßt.

Tabelle III

| Bsp.-Nr. | Dosis kg a.i./ha | herbizide Wirkung SIA | CRS | LOM | ECG |
|---|---|---|---|---|---|
| 1 | 2,5 | 5 | 5 | 5 | 5 |
| 2 | 2,5 | 5 | 5 | 5 | 5 |
| 3 | 2,5 | 5 | 5 | 5 | 5 |
| 8 | 2,5 | 5 | 4 | 5 | 5 |
| 20 | 2,5 | 4 | 4 | 5 | 3 |
| 23 | 2,5 | 4 | 2 | 4 | 5 |
| 81 | 2,5 | 5 | 5 | 5 | 5 |
| 82 | 2,5 | 5 | 3 | 4 | 4 |
| 50 | 2,5 | 4 | 3 | 4 | 4 |
| 54 | 2,5 | 5 | 2 | 5 | 5 |
| 56 | 2,5 | 5 | 5 | 5 | 5 |
| 58 | 2,5 | 5 | 4 | 5 | 5 |
| 97 | 2,5 | 4 | 2 | 2 | 1 |
| 106 | 2,5 | 5 | 5 | 5 | 5 |
| 134 | 2,5 | 4 | 4 | 4 | 4 |
| 146 | 2,5 | 5 | 5 | 5 | 5 |
| 168 | 2,5 | 5 | 2 | 5 | 4 |
| 176 | 2,5 | 5 | 4 | 5 | 5 |

3. Kulturpflanzenverträglichkeit

In weiteren Versuchen im Gewächshaus wurden Samen einer größeren Anzahl von Kulturpflanzen in sandigem Lehmboden ausgelegt und mit Erde abgedeckt. Ein Teil der Töpfe wurde sofort wie unter 1. beschrieben behandelt; die übrigen wurden im Gewächshaus aufgestellt, bis die Pflanzen zwei bis drei/echte Blät ter entwickelt hatten und dann mit den erfindungsgemäßen Substanzen in unterschiedlichen Dosierungen, wie unter 2. beschrieben, besprüht.

Vier bis fünf Wochen nach der Applikation und Standzeit im Gewächshaus wurde mittels optischer Bonitur festgestellt, daß die erfindungsgemäßen Verbindungen zweikeimblättrige Kulturen wie z. B. Soja, Baumwolle, Raps, Zuckerrüben und Kartoffeln im Vor-und Nachauf-laufverfahren selbst bei hohen Wirkstoffdosierungen ungeschädigt ließen. Einige Substanzen schonten darüber hinaus auch Gramineen-Kulturen wie z. B. Gerste, Sorghum, Mais, Weizen oder Reis. Die Verbindungen der Formel I weisen somit eine hohe Selektivität bei Anwendung zur Bekämpfung von unerwünschtem Pflanzenwuchs in landwirtschaftlich wichtigen Kulturen auf.

## Ansprüche

1. Herbizide Mittel, dadurch gekennzeichnet, daß sie eine Verbindung der Formel I

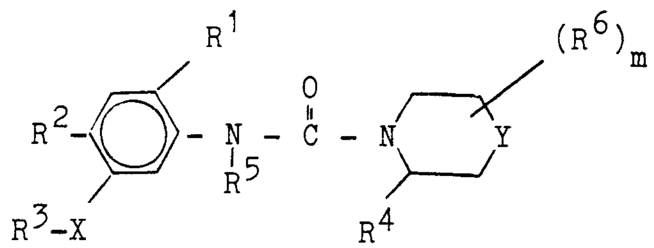

(I),

worin

X = Sauerstoff, Schwefel oder eine direkte Bindung

$R^1$ = Wasserstoff, Fluor oder Chlor,

$R^2$ = Fluor, Chlor, Brom, ($C_1$-$C_4$)Alkyl, das bis zu 6-fach durch Fluor und/oder Chlor substituiert sein kann, oder ($C_1$-$C_4$)Alkoxy, das bis zu 6-fach durch Fluor und/oder Chlor substituiert sein kann

$R^3$ = Wasserstoff, ($C_1$-$C_4$)Alkyl, ($C_3$-$C_6$)Cycloalkyl, ($C_3$-$C_6$)Alkenyl, ($C_5$-$C_6$)Cycloalkenyl, ($C_3$-$C_6$)-Alkinyl, ($C_1$-$C_4$)-Alkoxycarbonyl, ($C_1$-$C_4$)Alkoxycarbonyl($C_1$-$C_4$)-alkoxycarbonyl, ($C_3$-$C_4$)Alkinyloxycarbonyl($C_1$-$C_4$)-alkoxycarbonyl, ($C_1$-$C_4$)Alkoxy($C_1$-$C_4$)alkoxycarbonyl-($C_1$-$C_4$)alkoxycarbonyl, Trifluorethoxycarbonyl, Trifluorethoxycarbonyl($C_1$-$C_4$)alkoxycarbonyl, ($C_1$-$C_4$)Alkoxy($C_1$-$C_4$)alkyl, ($C_1$-$C_4$)-Alkoxy-carbonyl-($C_1$-$C_4$)alkyl, ($C_1$-$C_4$)Alkoxythiocarbonyl($C_1$-$C_4$)alkyl, Phenyl oder Benzyl, die beide bis zu dreifach im Phenylring durch Halogen, ($C_1$-$C_4$)Alkyl, ($C_1$-$C_4$)-Alkoxy, ($C_1$-$C_4$)-Alkoxy-carbonyl, $CF_3$, $NO_2$ oder CN substituiert sein können,

$R^4$ = -CN, 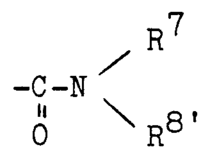, 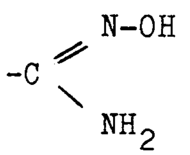, $-CO-ZR^3$,

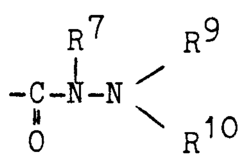 oder 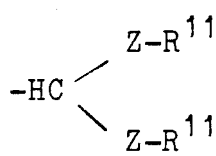

Y = -O-, 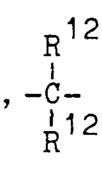, -S-, 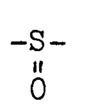, 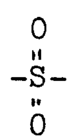 oder 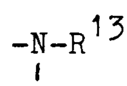

Z = Sauerstoff oder Schwefel,

$R^5$ Wasserstoff, $(C_1$-$C_4)$Alkyl, $(C_1$-$C_4)$Alkoxycarbonyl-$(C_1$-$C_4)$alkyl oder Hydroxymethyl,

$R^6$ $(C_1$-$C_4)$Alkyl, $(C_1$-$C_4)$Alkoxycarbonyl, $(C_1$-$C_4)$Alkoxycarbonyl$(C_1$-$C_4)$alkoxycarbonyl,Phenyl oder Benzyl, die beide bis zu 3-fach durch Halogen, $(C_1$-$C_4)$Alkyl, $(C_1$-$C_4)$Alkoxy, $CF_3$ oder $NO_2$ substituiert sein können,

$R^7$,$R^8$ unabhängig voneinander Wasserstoff, $(C_1$-$C_4)$Alkoxy, Benzyloxy, $(C_1$-$C_4)$-Alkyl, $(C_3$-$C_6)$Cycloalkyl, $(C_3$-$C_6)$Alkenyl, $(C_5$-$C_6)$Cycloalkenyl, $(C_3$-$C_6)$Alkinyl, Phenyl oder Benzyl, die beide im Phenylring ein-bis dreifach durch Halogen, $(C_1$-$C_4)$Alkyl, $(C_1$-$C_4)$-Alkoxy, $(C_1$-$C_4)$Alkoxy-carbonyl, $CF_3$, CN oder $NO_2$ substituiert sein können, oder

$R^7$ und $R^8$ zusammen mit dem Stickstoffatom, an das sie gebunden sind, einen fünf-bis sechsgliedrigen, gesättigten, gegebenenfalls bis zu vierfach durch $(C_1$-$C_4)$Alkyl substituierten, heterocyclischen Ring bilden, der ein oder mehrere Ringglieder aus der Gruppe O, S, $NR^{14}$, wobei $R^{14}$ Wasserstoff, $(C_1$-$C_4)$-Alkyl, Phenyl oder Benzyl, die beide ein-bis dreifach durch Halogen, $CF_3$, $(C_1$-$C_4)$-Alkyl oder $(C_1$-$C_4)$-Alkoxy substituiert sein können, bedeutet, enthält und worin zusätzlich eine Methylengruppe durch C=O ersetzt sein kann, und der Ring zusätzlich einen ankondensierten Benzolring oder einen ankondensierten 5-bis 6-gliedrigen gesättigten Kohlenwasserstoffring aufweisen kann,

$R^9$, $R^{10}$ unabhängig voneinander Wasserstoff, $(C_1$-$C_4)$Alkyl, Phenyl oder Benzyl, die beide bis zu 3-fach durch $(C_1$-$C_4)$Alkyl, Halogen, $CF_3$, $NO_2$ oder $(C_1$-$C_4)$Alkoxy substituiert sein können; $(C_1$-$C_4)$Alkoxycarbonyl; oder einer der Reste $R^9$ oder $R^{10}$ einen Rest der Formeln -CO-$R^{15}$ , -CO-$NHR^{15}$ , -$S(O)_nR^{15}$ oder $-\underset{Z}{\overset{\|}{P}}(ZR^{15})_2$ oder

$R^9$ und $R^{10}$ zusammen die Gruppe $=C(R^{15})_2$ ,

$R^{11}$ jeweils $(C_1$-$C_4)$Alkyl oder zwei Reste $R^{11}$ zusammen mit dem Rest

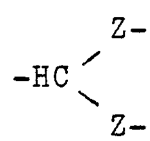

an den sie gebunden sind, einen 5-oder 6-gliedrigen gesättigten Heterocyclus, der bis zu 2-fach durch $(C_1$-$C_4)$Alkyl substituiert sein kann,

$R^{12}$ unabhängig voneinander Wasserstoff, $(C_1$-$C_4)$Alkyl, $(C_1$-$C_4)$Alkoxy-Carbonyl, Phenyl oder Benzyl, die beide bis zu 3-fach durch $(C_1$-$C_4)$Alkoxycarbonyl, $(C_1$-$C_4)$Alkyl, $(C_1$-$C_4)$Alkoxy oder Halogen substituiert sein können,

$R^{13}$ Wasserstoff, $(C_1$-$C_4)$Alkyl, Phenyl oder Benzyl, die beide bis zu 3-fach durch Halogen, $(C_1$-$C_4)$Alkyl, $(C_1$-$C_4)$Alkoxy, $CF_3$ oder $NO_2$ substituiert sein können, einen Rest der Formeln -CO-$R^{15}$ , -CO-$NHR^{15}$ , -CO-$ZR^{15}$ , -$S(O)_nR^{15}$ oder $-\overset{Z}{\underset{\|}{P}}(ZR^{15})_2$ ,

$R^{15}$ unabhängig voneinander Wasserstoff, $(C_1$-$C_4)$Alkyl, Phenyl oder Benzyl, die beide bis zu 3-fach durch Halogen, $(C_1$-$C_4)$Alkyl, $(C_1$-$C_4)$Alkoxy, $CF_3$ oder $NO_2$ substituiert sein können, und

m,n = 0, 1, 2, 3 bedeuten,

mit der Maßgabe, daß wenn X = eine direkte Bindung, m = 0, $R^4$ = $(C_1$-$C_4)$Alkoxycarbonyl, $R^{15}$ = H und Y = $CH_2$ bedeutet, $R^3$ nicht $(C_1$-$C_4)$Alkoxycarbonyl und $(C_1$-$C_4)$Alkoxy$(C_1$-$C_4)$-alkoxycarbonyl sein darf, enthalten.

2. Herbizide Mittel gemäß Anspruch 1, dadurch gekennzeichnet, daß X = 0 oder S, $R^1$ = H oder Fluor, Y = $C(R^{12})_2$-, 0 oder S, $R^5$ = H oder $(C_1$-$C_4)$Alkyl, $R^6$ = $(C_1$-$C_4)$Alkyl, $(C_1$-$C_4)$Alkoxycarbonyl oder $(C_1$-$C_4)$-Alkoxycarbonyl$(C_1$-$C_4)$-alkoxycarbonyl und m = 0 oder 1 bedeuten.

3. Verwendung von Verbindungen der Formel I oder deren Salze als Herbizide.

4. Verfahren zur Bekämpfung von unerwünschten Pflanzen, dadurch gekennzeichnet, daß man eine wirksame Menge einer Verbindung der Formel I oder deren Salze auf die zu behandelnden Anbauflächen bzw. die zu behandelnden Pflanzen aufbringt.

5. Verbindungen der Formel I von Anspruch 1 und deren Salze mit Ausnahme der Verbindungen der Formel I, worin $R^1$ = F, $R^2$ = Cl oder Br, X = Sauerstoff, Y = $CH_2$, S oder $SO_2$, $R^3$ = $(C_1$-$C_4)$Alkyl, Allyl, oder Propargyl und $R^4$ = -$COOR^{3'}$ , mit $R^3$ = Alkyl oder ein Alkalimetallatom, bedeuten.

6. Verfahren zur Herstellung von Verbindungen der Formel I und deren Salze gemäß Anspruch 5, dadurch gekennzeichnet, daß man

a) ein Isocyanat der Formel II

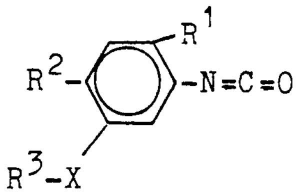

(II)

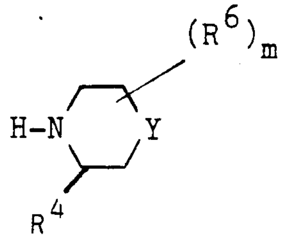

(III)

mit einer Verbindung der Formel III gegebenenfalls in Gegenwart einer Base, oder
b) eine Verbindung der Formel IV mit einer Verbindung der Formel V

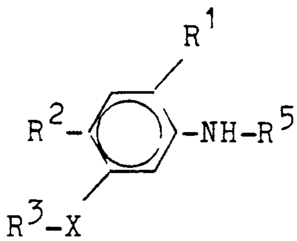

(IV)

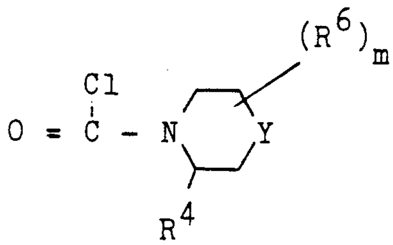

(V)

in Gegenwart einer Base oder
c) eine Verbindung der Formel VI

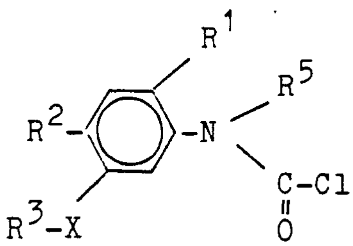

(VI)

mit einer Verbindung der Formel III
in Gegenwart einer Base umsetzt und die unter a) bis c) erhaltenen Verbindungen der Formel I im Falle $R^4$ = -CO-ZH gegebenenfalls in ihre Salze überführt.

Patentansprüche für folgenden Verstragsstaten : AT; ES

1. Herbizide Mittel, dadurch gekennzeichnet, daß sie eine Verbindung der Formel I

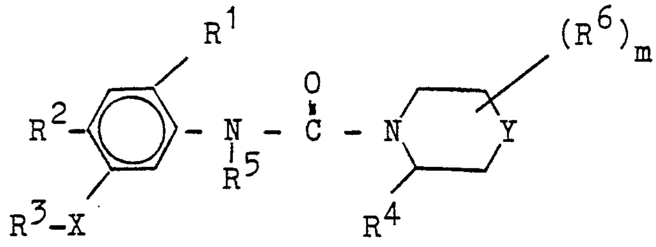 (I),

worin

X = Sauerstoff, Schwefel oder eine direkte Bindung

$R^1$ = Wasserstoff, Fluor oder Chlor,

$R^2$ = Fluor, Chlor, Brom, $(C_1-C_4)$Alkyl, das bis zu 6-fach durch Fluor und/oder Chlor substituiert sein kann, oder $(C_1-C_4)$Alkoxy, das bis zu 6-fach durch Fluor und/oder Chlor substituiert sein kann

$R^3$ = Wasserstoff, $(C_1-C_4)$Alkyl, $(C_3-C_6)$Cycloalkyl, $(C_3-C_6)$Alkenyl, $(C_5-C_6)$Cycloalkenyl, $(C_3-C_6)$-Alkinyl, $(C_1-C_4)$-Alkoxycarbonyl, $(C_1-C_4)$Alkoxycarbonyl$(C_1-C_4)$-alkoxycarbonyl, $(C_3-C_4)$Alkinyloxycarbonyl$(C_1-C_4)$-alkoxycarbonyl, $(C_1-C_4)$Alkoxy$(C_1-C_4)$alkoxycarbonyl-$(C_1-C_4)$alkoxycarbonyl, Trifluorethoxycarbonyl, Trifluorethoxycarbonyl$(C_1-C_4)$alkoxycarbonyl, $(C_1-C_4)$Alkoxy$(C_1-C_4)$alkyl, $(C_1-C_4)$-Alkoxy-carbonyl-$(C_1-C_4)$alkyl, $(C_1-C_4)$Alkoxythiocarbonyl$(C_1-C_4)$alkyl, Phenyl oder Benzyl, die beide bis zu dreifach im Phenylring durch Halogen, $(C_1-C_4)$Alkyl, $(C_1-C_4)$-Alkoxy, $(C_1-C_4)$-Alkoxy-carbonyl, $CF_3$, $NO_2$ oder CN substituiert sein können,

$R^4$ = -CN, 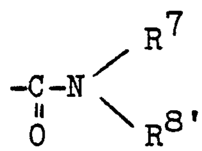, 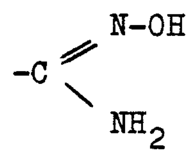, $-CO-ZR^3$,

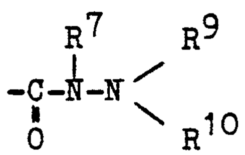 oder 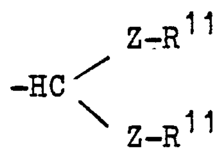

Y = 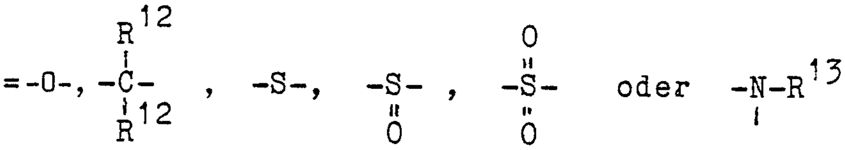

Z = Sauerstoff oder Schwefel,

$R^5$ Wasserstoff, $(C_1-C_4)$Alkyl, $(C_1-C_4)$Alkoxycarbonyl-$(C_1-C_4)$alkyl, oder Hydroxymethyl,

$R^6$ $(C_1-C_4)$Alkyl, $(C_1-C_4)$Alkoxycarbonyl, $(C_1-C_4)$Alkoxycarbonyl$(C_1-C_4)$alkoxycarbonyl, Phenyl oder Benzyl, die beide bis zu 3-fach durch Halogen, $(C_1-C_4)$Alkyl, $(C_1-C_4)$Alkoxy, $CF_3$ oder $NO_2$ substituiert sein können,

$R^7$,$R^8$ unabhängig voneinander Wasserstoff, $(C_1-C_4)$Alkoxy, Benzyloxy, $(C_1-C_4)$-Alkyl, $(C_3-C_6)$Cycloalkyl, $(C_3-C_6)$Alkenyl, $(C_5-C_6)$Cycloalkenyl, $(C_3-C_6)$Alkinyl, Phenyl oder Benzyl, die beide im Phenylring ein-bis dreifach durch Halogen, $(C_1-C_4)$Alkyl, $(C_1-C_4)$-Alkoxy, $(C_1-C_4)$Alkoxy-carbonyl, $CF_3$, CN oder $NO_2$ substituiert sein können, oder

$R^7$ und $R^8$ zusammen mit dem Stickstoffatom, an das sie gebunden sind, einen fünf-bis sechsgliedrigen, gesättigten, gegebenenfalls bis zu vierfach durch $(C_1-C_4)$Alkyl substitutierten, heterocyclischen Ring bilden,

der ein oder mehrere Ringglieder aus der Gruppe O, S, $NR^{14}$, wobei $R^{14}$ Wasserstoff, $(C_1$-$C_4)$-Alkyl, Phenyl oder Benzyl, die beide ein-bis drei-fach durch Halogen, $CF_3$, $(C_1$-$C_4)$-Alkyl oder $(C_1$-$C_4)$-Alkoxy substituiert sein können, bedeutet, enthält und worin zusätzlich eine Methylengruppe durch C = O ersetzt sein kann, und der Ring zusätzlich einen ankondensierten Benzolring oder einen ankondensierten 5-bis 6-gliedrigen gesättigten Kohlenwasserstoffring aufweisen kann,

$R^9$, $R^{10}$ unabhängig voneinander Wasserstoff, $(C_1$-$C_4)$Alkyl, Phenyl oder Benzyl, die beide bis zu 3-fach durch $(C_1$-$C_4)$Alkyl, Halogen, $CF_3$, $NO_2$ oder $(C_1$-$C_4)$Alkoxy substituiert sein können; $(C_1$-$C_4)$Alkoxycarbonyl; oder einer der Reste $R^9$ oder $R^{10}$ einen Rest der Formeln $-CO-R^{15}$, $-CO-NHR^{15}$, $-S(O)_nR^{15}$ oder $-\overset{Z}{P}(ZR^{15})_2$ oder

$R^9$ und $R^{10}$ zusammen die Gruppe $=C(R^{15})_2$,

$R^{11}$ jeweils $(C_1$-$C_4)$Alkyl oder zwei Reste $R^{11}$ zusammen mit dem Rest

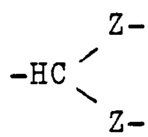

an den sie gebunden sind, einen 5-oder 6-gliedrigen gesättigten Heterocyclus, der bis zu 2-fach durch $(C_1$-$C_4)$Alkyl substituiert sein kann,

$R^{12}$ unabhängig voneinander Wasserstoff, $(C_1$-$C_4)$Alkyl, $(C_1$-$C_4)$Alkoxy-Carbonyl, Phenyl oder Benzyl, die beide bis zu 3-fach durch $(C_1$-$C_4)$Alkoxycarbonyl,$(C_1$-$C_4)$Alkyl, $(C_1$-$C_4)$Alkoxy oder Halogen substituiert sein können,

$R^{13}$ Wasserstoff, $(C_1$-$C_4)$Alkyl, Phenyl oder Benzyl, die beide bis zu 3-fach durch Halogen, $(C_1$-$C_4)$Alkyl, $(C_1$-$C_4)$Alkoxy, $CF_3$ oder $NO_2$ substituiert sein können, einen Rest der Formeln $-CO-R^{15}$, $-CO-NHR^{15}$, $-CO-ZR^{15}$, $-S(O)_nR^{15}$ oder $-\overset{Z}{P}(ZR^{15})_2$,

$R^{15}$ unabhängig voneinander Wasserstoff, $(C_1$-$C_4)$Alkyl, Phenyl oder Benzyl, die beide bis zu 3-fach durch Halogen, $(C_1$-$C_4)$Alkyl, $(C_1$-$C_4)$Alkoxy, $CF_3$ oder $NO_2$ substituiert sein können, und

m,n = 0, 1, 2, 3 bedeuten,

mit der Maßgabe, daß wenn X = eine direkte Bindung, m = 0,

$R^4$ = $(C_1$-$C_4)$Alkoxycarbonyl, $R^{15}$ = H und Y = $CH_2$ bedeutet,

$R^3$ nicht $(C_1$-$C_4)$Alkoxycarbonyl und $(C_1$-$C_4)$Alkoxy$(C_1$-$C_4)$-alkoxycarbonyl sein darf, enthalten.

2. Herbizide Mittel gemäß Anspruch 1, dadurch gekennzeichnet, daß X = 0 oder S, $R^1$ = H oder Fluor, Y = $C(R^{12})_2$-, 0 oder S, $R^5$ = H oder $(C_1$-$C_4)$Alkyl, $R^6$ = $(C_1$-$C_4)$Alkyl, $(C_1$-$C_4)$Alkoxycarbonyl oder $(C_1$-$C_4)$-Alkoxycarbonyl$(C_1$-$C_4)$-alkoxycarbonyl und m = 0 oder 1 bedeuten.

3. Verwendung von Verbindungen der Formel I oder deren Salze als Herbizide.

4. Verfahren zur Bekämpfung von unerwünschten Pflanzen, dadurch gekennzeichnet, daß man eine wirksame Menge einer Verbindung der Formel I oder deren Salze auf die zu behandelnden Anbauflächen bzw. die zu behandelnden Pflanzen aufbringt.

Europäisches Patentamt

# EUROPÄISCHER RECHERCHENBERICHT

Nummer der Anmeldung

EP 86114577.9

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,A | EP - A1 - 0 104 532 (NIPPON KAYAKU KABUSHIKI KAISHA)<br>* Ansprüche 1,7,8,10,11,13 *<br>-- | 1-6 | A 01 N 47/38<br>C 07 D 211/60<br>C 07 D 211/62<br>C 07 D 211/64<br>C 07 D 265/30<br>C 07 D 279/12<br>C 07 D 405/04 |
| D,A | EP - A2/A3 - 0 070 389 (SUMITOMO CHEMICAL COMPANY, LIMITED)<br>* Ansprüche 9,16 *<br>-- | 5,6 | |
| A | DE - A1 - 2 532 645 (CONSORTIUM FÜR ELEKTROCHEMISCHE INDUSTRIE GMBH)<br>* Anspruch 1; Seite 4 *<br>-- | 1-6 | |
| A | DE - A1 - 2 416 139 (CONSORTIUM FÜR ELEKTROCHEMISCHE INDUSTRIE GMBH)<br>* Anspruch; Seite 3 *<br>-- | 1-6 | **RECHERCHIERTE SACHGEBIETE (Int. Cl.4)** |
| A | DE - B - 1 188 861 (BADISCHE ANILIN- & SODA-FABRIK AG)<br>* Anspruch; Spalte 1 *<br>-- | 1-6 | A 01 N 47/00<br>C 07 D 211/00<br>C 07 D 265/00<br>C 07 D 279/00<br>C 07 D 405/00 |
| A | US - A - 4 519 834 (H.SZCZEPANSKI)<br>* Zusammenfassung; Spalten 3,4 *<br>---- | 1-6 | |

Der vorliegende Recherchenbericht wurde für alle Patentansprüche erstellt.

| Recherchenort | Abschlußdatum der Recherche | Prüfer |
|---|---|---|
| WIEN | 28-01-1987 | PETROUSEK |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82